# EUROPEAN PATENT APPLICATION

(11) **EP 4 559 462 A1**
(43) Date of publication of application: **28.05.2025**
(21) Application number: 23843355.1
(22) Date of filing: 19.07.2023
(51) Int. Cl.: A61K 31/405, A61P 3/04, A61P 3/10, A61P 1/16, A61P 3/06, A61P 9/00, A23L 33/10, A23K 20/132

(54) **USE OF PPAR AGONIST FOR IMPROVEMENT OF ENERGY METABOLISM**

(30) Priority: 19.07.2022 KR 20220089073
(71) Applicant: Novmetapharma Co., Ltd., Seoul 06050 (KR); POSTECH Research and Business Development Foundation, Pohang-si, Gyeongsangbuk-do 37673 (KR)
(72) Inventor: KIM, Kyong Tai, Pohang-si Gyeongsangbuk-do 37673 (KR); OH, Eunji, Seoul 02543 (KR); KANG, Jeonghwa, Jeonju-si Jeollabuk-do 55049 (KR); CHO, Sungji, Seoul 07296 (KR); JUNG, Hoe Yune, Pohang-si Gyeongsangbuk-do 37668 (KR); LEE, Heon Jong, Incheon 22760 (KR)
(74) Representative: Marks & Clerk LLP
(86) International application number: PCT/KR2023/010378
(87) International publication number: WO 2024/019509

(57) **Abstract**

The present invention provides a pharmaceutical composition for use in preventing or treating metabolic diseases comprising a novel pan-PPAR agonist compound or a salt thereof as an active ingredient. As the pan-PPAR agonist, the compound or the salt thereof may be useful in pharmaceutical compositions for preventing or treating metabolic diseases such as obesity, diabetes, fatty liver diseases, dyslipidemia, cardiovascular diseases, and/or metabolic syndrome, and in food or feed compositions for improving metabolic health.

## Description

### [Technical Field]

This invention claims the benefit of priority from Korean Patent Application No. 10-2022-0089073, the entire contents of which are incorporated herein by reference.

The present invention relates to the use of a novel pan-PPAR agonist for preventing, improving or treating metabolic disorders.

### [Background Art]

Peroxisome proliferator-activated receptors (PPARs) are nuclear hormone receptor family proteins that contain PPAR-responsive regulatory elements (PPREs) together with retinoid X receptors (RXR) and may control gene transcription, and are classified into isotypes such as PPARα, PPARβ/δ, and PPARγ.

PPAR acts as a lipid sensor within cells and functions as a key factor in normal energy metabolism homeostasis. The PPAR agonists may improve various energy metabolism homeostasis conditions such as obesity, lipid metabolism, glucose homeostasis, and insulin resistance, and are considered as major targets for metabolic disorders (*see* Corrales, Patricia, Antonio Vidal-Puig, and Gema Medina-Gomez. International Journal of Molecular Sciences 19.7 (2018): 2124., etc.).

Specifically, PPARα is involved in increasing fatty acid uptake, esterification, and trafficking in cells, regulates lipoprotein metabolism genes, and is activated by (8S)-hydroxyeicosatetraenoic acid, pemafibrate (K-877), fenofibrate, WY14643, etc. (*see* Sasaki, Yusuke, et al. Scientific reports 10.1 (2020): 1-10, etc.).

PPARβ/δ promotes lipid and glucose utilization by increasing a mitochondrial function and a fatty acid desaturation pathway, and is activated by (13S)-hydroxyoctadecadienoic acid, GW501516, seladelpar (MBX-8025), L-165041, etc. (*see* Li, Xiuli, et al. International Journal of Molecular Medicine 36.3 (2015): 767-775., etc.).

PPARγ increases insulin sensitivity and glucose metabolism by promoting fatty acid uptake, triglyceride formation, and lipid droplet storage, and is activated by pioglitazone, rosiglitazone, etc. (*see* Soccio, Raymond E., Eric R. Chen, and Mitchell A. Lazar. Cell metabolism 20.4 (2014): 573-591.).

In addition, in order to improve PPAR agonists, dual PPARα and PPARγ agonists such as saroglitazar and tesaglitazar (*see* Rastogi, Ashu, et al. Acta Diabetologica 57.7 (2020): 809-818., etc.), dual PPARα and PPARβ/δ agonists such as elafibranor (GFT505) (*see* Ratziu, Vlad, et al. Gastroenterology 150.5 (2016): 1147-1159., etc.), and PPARα, PPARβ/δ, and PPARγ pan-agonists such as lanifibranor and chiglitazar have been developed (*see* Lefere, Sander, et al. Journal of hepatology 73.4 (2020): 757-770., etc.), there is a need to develop new PPAR agonists to improve the efficacy and side effects of conventional PPAR agonists.

### [Disclosure]

### [Technical Problem]

Accordingly, the present inventors discovered a novel PPAR agonist capable of improving metabolic disorders such as obesity, diabetes, and fatty liver diseases, and confirmed that the PPAR agonist effectively improved various metabolic disorders in cell and animal models, and then completed the present invention.

An object of the present invention is to provide the use of a compound represented by Chemical Formula 1 or a salt thereof for preventing, improving or treating metabolic disorders.

Another object of the present invention is to provide the use of a compound represented by Chemical Formula 1 or a salt thereof as a PPAR agonist.

### [Technical Solution]

In one aspect, the present invention provides a pharmaceutical composition for use in preventing or treating metabolic diseases, including a compound represented by the following Chemical Formula 1 or a salt thereof as an active ingredient.

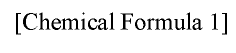

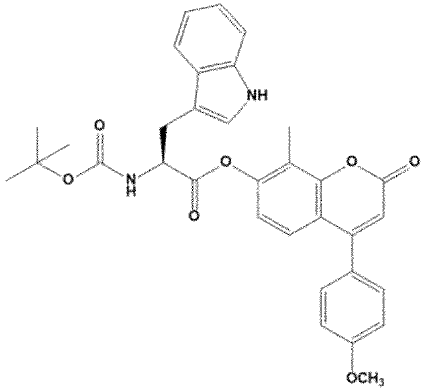

The compound represented by Chemical Formula 1 or the salt thereof may activate PPARα, PPARβ/δ, and PPARγ in vivo.

The metabolic disease may be at least one selected from the group consisting of obesity, diabetes, fatty liver diseases, metabolic syndrome, dyslipidemia, and cardiovascular diseases.

In another aspect, the present invention provides a health functional food composition for use in improving metabolic health including a compound represented by Chemical Formula 1 or a salt thereof.

The improvement of the metabolic health may be at least one effect selected from the group consisting of improvement in obesity, drop in blood sugar, improvement in fatty liver, improvement in lipid metabolism, improvement in blood circulation, and improvement in metabolic syndrome.

In yet another aspect, the present invention provides a feed composition for use in improving metabolic health including a compound represented by Chemical Formula 1 or a salt thereof.

In yet another aspect, the present invention provides a method for activating a PPAR protein, including treating a compound represented by Chemical Formula 1 or a salt thereof to an isolated cell. The PPAR protein may include at least one selected from the group consisting of PPARα, PPARβ/δ, and PPARγ.

### [Advantageous Effects]

According to the present invention, the compound represented by Chemical Formula 1 or the salt thereof is the pan-PPAR agonist, and can be useful in a pharmaceutical composition for use in preventing or treating metabolic diseases such as obesity, diabetes, fatty liver diseases, dyslipidemia, cardiovascular diseases, and/or metabolic syndrome, and in food or feed compositions for improving metabolic health.

### [Description of Drawings]

FIG. 1 shows results of measuring changes in luciferase activity after drug treatment in HEK293 cells transfected with a Gal4-luciferase reporter system.
FIG. 2 shows results of pull-down analysis of PPAR proteins using CNBr beads conjugated with drugs.
FIG. 3 shows Oil Red O staining photographs of drug-treated differentiated adipocytes (scale bar 100 µm, magnification 200X).
FIG. 4 shows results of quantifying lipid accumulation (left) and lipid droplet size distribution (right) in adipocytes stained with Oil Red O.
FIG. 5 shows an animal experiment schedule for evaluating effects of drug administration.
FIG. 6 shows results of measuring body weight change (left), body weight (middle), and food intake (right) in high-fat diet mice administered with drugs.
FIG. 7 shows results of micro-CT imaging of high-fat diet mice administered with drugs (adipose tissue: red, muscle: yellow).
FIG. 8 shows results of measuring adipose tissue and muscle volumes in high-fat diet mice administered with drugs.
FIG. 9 shows results of measuring organ weights in high-fat diet mice administered with drugs.
FIG. 10 shows results of an intraperitoneal glucose tolerance test (IP-GTT) in high-fat diet mice administered with drugs.
FIG. 11 shows results of an intraperitoneal insulin tolerance test (IP-ITT) in high-fat diet mice administered with drugs.
FIG. 12 shows results of measuring serum glucose in high-fat diet mice administered with drugs.
FIG. 13 shows results of measuring serum lipids in high-fat diet mice administered with drugs.
FIG. 14 shows H&E staining images of adipose tissues in high-fat diet mice administered with drugs (scale bar 100 µm, magnification 200X).
FIG. 15 shows results of quantifying lipid droplet areas in adipose tissues of high-fat diet mice administered with drugs.
FIG. 16 shows H&E, Sirius Red, and Oil Red O staining images of liver tissues in high-fat diet mice administered with drugs (scale bar 100 µm, magnification 200X).
FIG. 17 shows results of measuring non-alcoholic fatty liver disease (NAFLD) activity scores in the liver tissues of high-fat diet mice administered with drugs.
FIG. 18 shows results of measuring the Oil red O staining areas in the liver tissues of high-fat diet mice administered with drugs.

### [Modes of the Invention]

Hereinafter, the present disclosure will be described in detail. Each description and embodiment disclosed in the present invention may also be applied to each of other descriptions and embodiments. That is, all combinations of various components disclosed in the present invention belong to the scope of the present invention. In addition, the specific description described below may not limit the scope of the present invention.

Further, those skilled in the art may recognize or determine a plurality of equivalents to specific embodiments of the present invention described in the present disclosure by using only a general experiment. In addition, such equivalents are intended to be included in the present invention.

In the present invention, a compound represented by Chemical Formula 1 is also referred to as [4-(4-methoxyphenyl)-8-methyl-2-oxochromen-7-yl] (2S)-3-(1H-indol-3-yl)-2-[(2-methylpropan-2-yl)oxycarbonylamino]propanoate (DTMB).

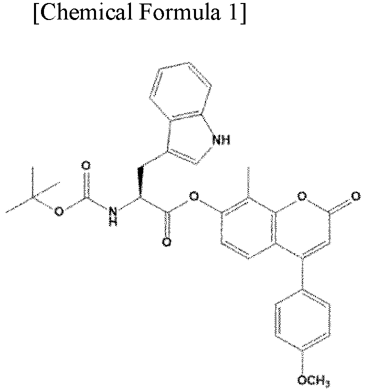

In the present invention, the salt of the compound represented by Chemical Formula 1 means a salt suitable for industrial application of the composition containing the salt, for example, a salt that is commonly usable in pharmaceuticals, quasi-drugs, foods, feeds, etc. The salt includes, for example, inorganic ion salts including sodium, potassium, calcium, magnesium, lithium, copper, manganese, zinc, iron, etc. of the compound represented by Chemical Formula 1; inorganic acid salts such as hydrochloric acid, phosphoric acid, and sulfuric acid; organic acid salts such as ascorbic acid, citric acid, tartaric acid, lactic acid, maleic acid, malonic acid, fumaric acid, glycolic acid, succinic acid, propionic acid, acetic acid, orotate acid, and acetylsalicylic acid; and amino acid salts such as lysine, arginine, and guanidine, but is not limited thereto.

In the present invention, the metabolic diseases mean metabolic disorders characterized by abnormal metabolism that may be caused by excessive nutrient intake, congenital enzyme abnormality, acquired endocrine abnormality, or loss of function of tissues involved in metabolism. In the present invention, the prevention or treatment of the metabolic diseases includes effects such as prevention, inhibition, delay, improvement, relief, alleviation, reduction, improvement, and treatment of the conditions or symptoms of metabolic diseases, and is not limited to complete suppression of the onset of metabolic diseases.

In the present invention, peroxisome proliferator-activated receptors (PPARs) are nuclear hormone receptor family proteins including isotypes such as PPARα, PPARβ/δ, and PPARγ, and are known to function as an intracellular lipid sensor to play a key role in regulating energy metabolism and homeostasis, and information on gene or protein sequences of human PPARα, PPARβ/δ, and PPARγ is known in the art.

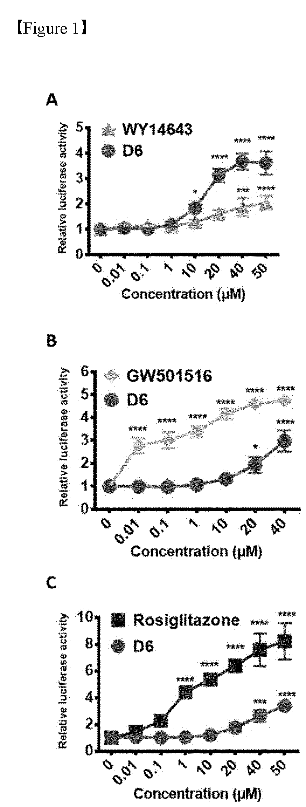

In an embodiment of the present invention, it was confirmed that the compound represented by Chemical Formula 1 was a pan-PPAR agonist capable of binding to PPARα, PPARβ/δ, and PPARγ to activate the transcription function of the PPAR proteins (FIG. 1). Therefore, the compound represented by Chemical Formula 1 or the salt thereof may be administered into the body to activate PPAR and improve energy metabolism, and thus may be useful for preventing or treating metabolic diseases.

In one embodiment, the metabolic disease is obesity. The obesity is a condition in which there is an excess of fat tissue in the body, and may be caused by energy imbalance when nutrients are consumed in excess for a long period of time compared to energy consumption. In addition, the obesity may also be caused by problems with the appetite center function due to specific genetic mutations, endocrine diseases, or the use of appetite-increasing drugs. The obesity may metabolically cause various obesity-related complications, such as diabetes, fatty liver, dyslipidemia, and cardiovascular diseases such as hypertension.

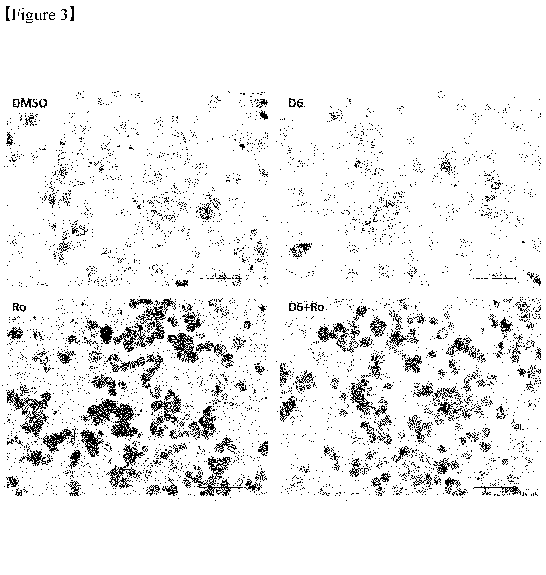

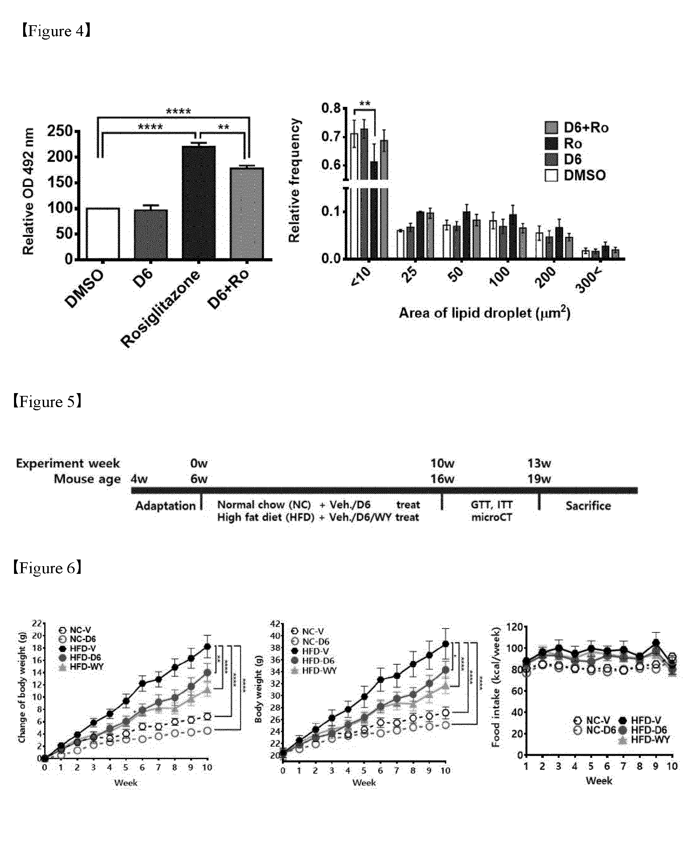

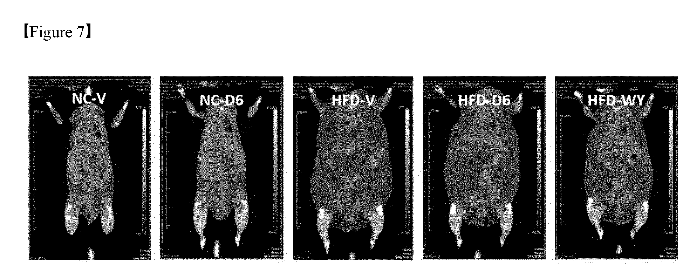

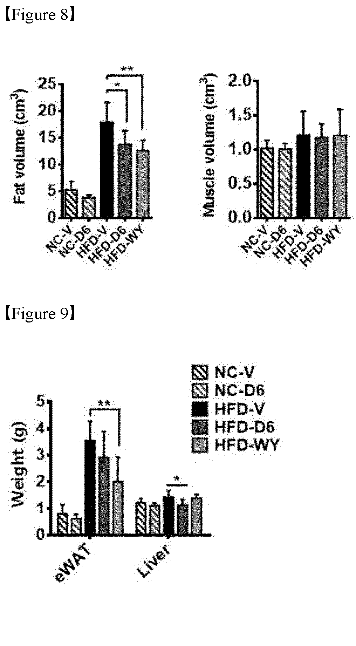

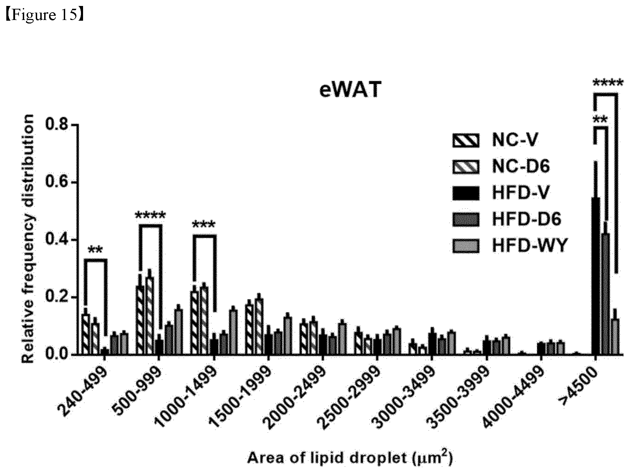

In an embodiment of the present invention, the compound represented by Chemical Formula 1 effectively inhibited fat accumulation in adipocytes (FIGS. 3 and 4), and was confirmed to have effects such as weight loss (FIG. 6), abdominal fat reduction (FIGS. 7 and 8), adipose tissue weight loss (FIG. 9), lipid droplet area reduction in adipose tissue (FIG. 15), and the like in a high-fat diet animal model. Therefore, the compound represented by Chemical Formula 1 or the salt thereof may be useful for the prevention or treatment of obesity, including fat reduction, weight loss, and obesity complications.

In one embodiment, the metabolic disease is diabetes. Diabetes mellitus is a type of metabolic disease characterized by hyperglycemia, in which insulin secretion is insufficient or insulin does not function normally, and may cause various diabetic complications, such as retinopathy, renal dysfunction, neuropathy, and cardiovascular disease, due to hyperglycemia. The diabetes is divided into type 1 diabetes, which is caused by abnormal insulin production, and type 2 diabetes, which is characterized by insulin resistance.

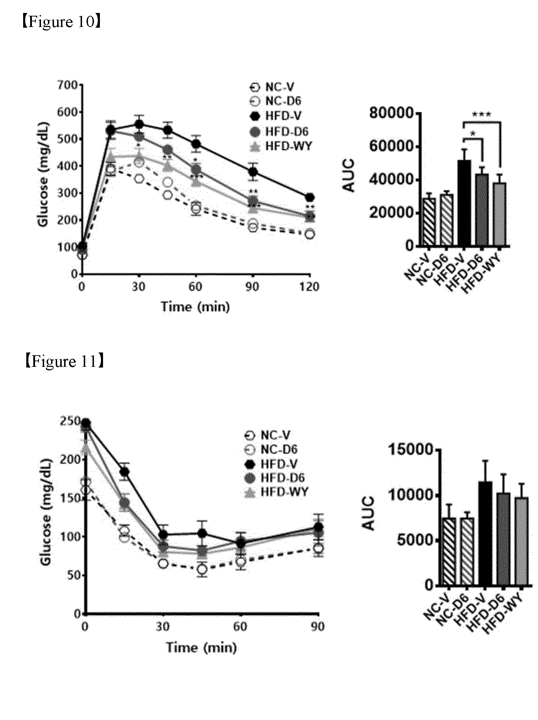

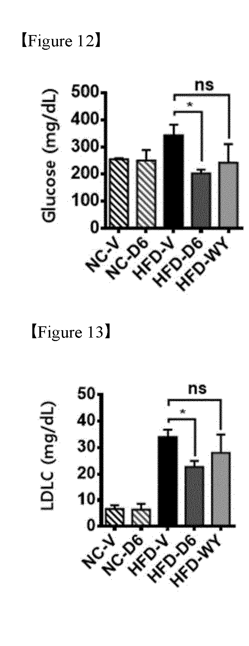

In an embodiment of the present invention, the compound represented by Chemical Formula 1 was confirmed to have effects, such as improved blood glucose tolerance (FIG. 10), improved insulin tolerance (FIG. 11), and drop in blood sugar (FIG. 12) in a high-fat diet animal model. Therefore, the compound represented by Chemical Formula 1 or the salt thereof may be useful for the prevention or treatment of diabetes, including hyperglycemia, insulin tolerance, impaired fasting glucose (IFG), impaired glucose tolerance (IGT), hyperinsulinemia, and diabetic complications.

In one embodiment, the metabolic disease is fatty liver disease. The fatty liver disease is a comprehensive concept that includes a condition in which fat accumulates in liver cells and the resulting liver tissue lesions. In the present invention, the fatty liver disease includes various sub-diseases depending on the degree of progression of the disease, ranging from simple fatty liver in which only fat is accumulated in the liver, steatohepatitis with inflammatory findings accompanied by hepatocyte damage (such as ballooning degeneration or fibrosis), and cirrhosis accompanied by steatohepatitis. In addition, depending on the cause of fatty liver disease, the fatty liver disease includes alcoholic fatty liver disease and nonalcoholic fatty liver disease. The nonalcoholic fatty liver disease (NAFLD) includes nonalcoholic fatty liver, nonalcoholic steatohepatitis (NASH), and nonalcoholic fatty liver disease-associated liver fibrosis and/or cirrhosis.

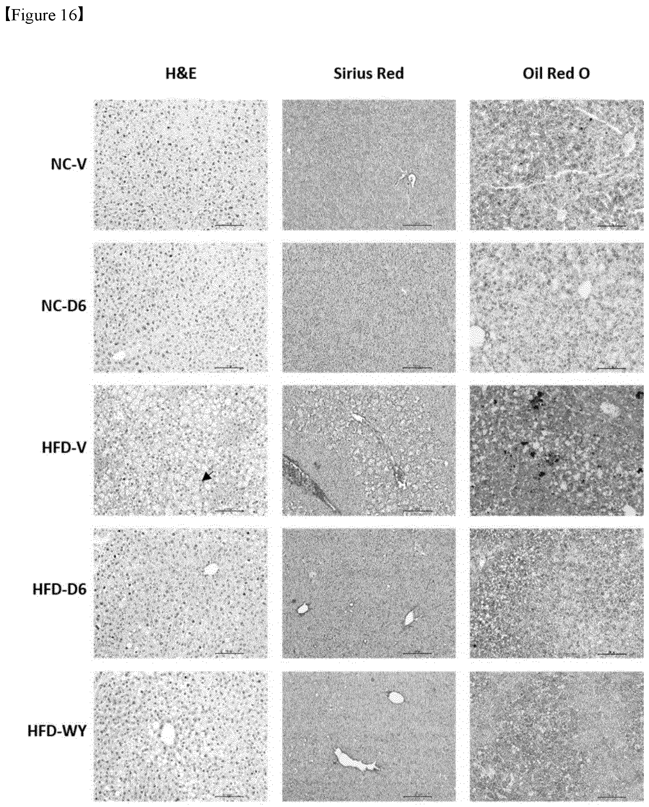

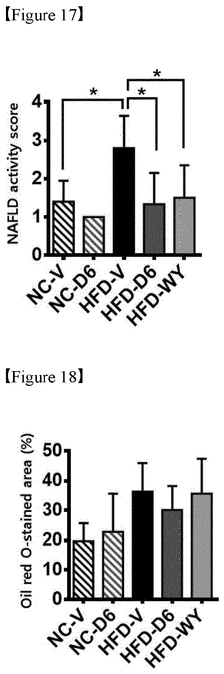

In an embodiment of the present invention, the compound represented by Chemical Formula 1 was confirmed to have effects such as reducing liver weight increased due to a high-fat diet in a high-fat diet animal model (FIG. 9), improving fatty liver findings in liver tissue (FIG. 16), reducing NAFLD activity score (FIG. 17), and reducing fat accumulation in liver tissue (FIG. 18). Therefore, the compound represented by Chemical Formula 1 or the salt thereof may be useful for the prevention or treatment of fatty liver disease, including reduced hepatic fat accumulation, improved hepatic inflammation, and improved hepatic fibrosis and/or cirrhosis.

In one embodiment, the metabolic disease is dyslipidemia. The dyslipidemia is a condition in which the normal concentration of lipids in the serum is increased or decreased, and is characterized by increased concentrations of total cholesterol, LDL cholesterol, and triglycerides in the blood, or decreased concentration of HDL cholesterol. The dyslipidemia may be caused by factors such as obesity or diabetes, or by an increase in specific lipids in the blood due to genetic factors. In addition, the dyslipidemia is a major risk factor for cardiovascular disease.

In an embodiment of the present invention, it was confirmed that the compound represented by Chemical Formula 1 effectively reduced LDL-cholesterol levels increased due to a high-fat diet in a high-fat diet animal model (FIG. 13). Therefore, the compound represented by Chemical Formula 1 or the salt thereof may be useful for the prevention or treatment of dyslipidemia including hyperlipidemia, hypercholesterolemia, hypertriglyceridemia, cardiovascular disease due to hyperlipidemia, xanthoma, lipoma, lipodystrophy, etc.

In one embodiment, the metabolic disease is cardiovascular disease. The metabolic disorders such as dyslipidemia and the like are major risk factors for cardiovascular disease, and the compound represented by Chemical Formula 1 or the salt thereof may be useful for the prevention or treatment of cardiovascular disease, for example, reduction of the risk of cardiovascular disease caused by dyslipidemia. The cardiovascular diseases include arteriosclerosis, atherosclerosis, hypertension, myocardial infarction, ischemic heart disease, stroke, and the like, but are not limited thereto.

In one embodiment, the metabolic disease is metabolic syndrome. The metabolic syndrome is a term that refers to a group of collecting various symptoms of metabolic diseases, and was first proposed as the term metabolic syndrome X, and is characterized by the simultaneous appearance of metabolic disorder symptoms such as obesity, hyperglycemia, hyperlipidemia, atherosclerosis, and hypertension. It is considered that insulin tolerance is a core cause of metabolic syndrome, and if the metabolic syndrome worsens, the risk of developing metabolic diseases such as fatty liver disease and cardiovascular disease may increase.

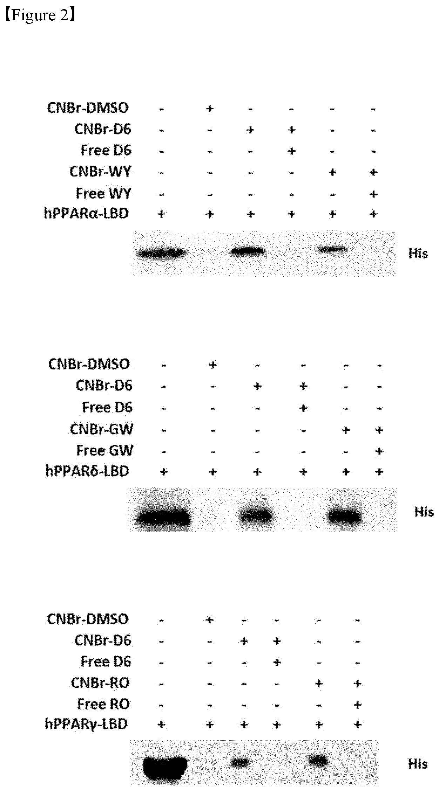

As described above, in the embodiment of the present invention, the effects of improving obesity, hyperglycemia, hyperlipidemia, etc. were confirmed in cell and animal models (FIGS. 2 to 18), and thus the compound represented by Chemical Formula 1 or the salt thereof may be useful for the prevention or treatment of metabolic syndrome.

The pharmaceutical composition of the present invention may include one or more pharmaceutically acceptable carriers, excipients, diluents, solubilizers, etc., in addition to the compound represented by Chemical Formula 1 or the salt thereof as active ingredients. The carriers, the excipients, and the diluents may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil, and the solubilizers may include poloxamer, labrasol, etc., but are not limited thereto.

The pharmaceutical composition may be present in various suitable formulations for oral or parenteral administration. In the present invention, the formulations for oral administration may include tablets, pills, powders, pulverized formulations, granules, pellets, capsules, troches, lozenges, suspensions, emulsions, syrups, elixirs, and the like, and the formulations for parenteral administration may include injections, suppositories, respiratory inhalants, aerosols, ointments, solutions, lotions, patches, powders for application, oils, creams, gels, and the like, but are not limited thereto.

The pharmaceutical composition may be administered orally or parenterally depending on the formulation. The parenteral administration may include subcutaneous administration, intradermal administration, transdermal administration, hair administration, intraperitoneal administration, rectal administration, intravenous administration, intramuscular administration, thoracic administration, and the like, but is not limited thereto.

The pharmaceutical composition may be administered in a pharmaceutically effective amount. Here, the pharmaceutically effective amount refers to an amount enough to treat diseases at a reasonable benefit/risk ratio applicable to medical treatment, and an effective amount level may be determined according to factors including the patient's condition, weight, sex, age, health condition, severity of disease, sensitivity to a drug, time of administration, route of administration, excretion rate, duration of treatment, concurrently used drugs, and other factors well known in the medical field.

The pharmaceutical composition further includes one or more active ingredients having an effect for preventing or treating metabolic diseases, in addition to the compound represented by Chemical Formula 1 or the salt thereof, and may be used as a combination agent for metabolic diseases.

The present invention also provides a method for preventing or treating metabolic diseases, including administering to a patient a compound represented by Chemical Formula 1 or a salt thereof in a therapeutically effective amount.

The present invention also provides the use of a compound represented by Chemical Formula 1 or a salt thereof for the prevention or treatment of metabolic diseases.

The present invention also provides the use of a compound represented by Chemical Formula 1 or a salt thereof for the prevention or treatment of metabolic diseases.

In the present invention, the food composition includes all food forms such as health foods, health functional foods, beverages, food additives, and food supplements, and may preferably be a health functional food. Here, the health functional foods refer to foods manufactured and processed using raw materials or ingredients that have functionality useful to the human body, so as to effectively exhibit bioregulatory functions in addition to providing nutrition.

The present invention also provides a method for improving metabolic health, including feeding a compound represented by Chemical Formula 1 or a salt thereof.

The present invention also provides the use of a compound represented by Chemical Formula 1 or a salt thereof for improving metabolic health.

The present invention also provides the use of a compound represented by Chemical Formula 1 or a salt thereof for the preparation of foods for improving metabolic health.

In the present invention, the feed composition is a diet provided for intake to animals including mammals other than humans, and may exhibit an effect of preventing, improving, or treating metabolic disorders of the animals.

The present invention also provides a method for improving metabolic health, including feeding a compound represented by Chemical Formula 1 or a salt thereof to animals other than humans.

The present invention also provides the use of a compound represented by Chemical Formula 1 or a salt thereof for improving metabolic health.

The present invention also provides the use of a compound represented by Chemical Formula 1 or a salt thereof for the preparation of feeds for improving metabolic health of animals other than humans.

Hereinafter, the present invention will be described in more detail by the following Examples. However, these Examples are only illustrative of the present invention, and the scope of the present invention is not limited to these Examples.

### <Materials and Methods>

### 1. Compounds and Reagents

A compound of the present invention represented by Chemical Formula 1 was synthesized by DAEJUNG Chemicals & Metals (Gyeonggi-do, Korea) (indicated as 'D6' in the drawing). WY14643, GW501516, and rosiglitazone were purchased from Sigma-Aldrich (St. Louis, MO, USA). The compounds were dissolved in dimethyl sulfoxide (DMSO), diluted in a culture solution, and used in the experiment. Cyanogen bromide (CNBr)-activated Sepharose 4B was purchased from Sigma-Aldrich (St. Louis, MO, USA). His-tagged recombinant human PPAR-ligand binding domain (LBD) was expressed in E. coli (DE3, Rosetta) and then purified and used. A His antibody was purchased from Cell Signaling Technology (Danvers, Massachusetts, USA). 3-Isobutyl-1-methylxanthine (IBMX), dexamethasone, bovine pancreatic insulin, and Oil Red O were purchased from Sigma-Aldrich (St. Louis, MO, USA). A PRO-PREP^{™} Protein Extraction Solution was purchased from iNtRON Biotechnology (iNtRON Biotech., Korea). Actin, HSL (Hormone Sensitive Lipase), and Adipose Triglyceride Lipase (ATGL) antibodies were purchased from Santa Cruz Biotechnology (Santa Cruz, CA, USA). Comet Assay Kits and 96-Well were purchased from Cell Biolabs (San Diego, CA, USA).

A plasmid was transformed into E. coli (DE3, Rosetta) and transfected into HEK293 cells and used. Referring to known literatures such as Kanno, Y. & Inouye, Y. J Toxicol Sci 35, 515-525 (2010), Zhao, S. et al. J Nat Prod 79, 879-885 (2016), etc., luciferase assay was performed on three types of pcDNA5-GAL4 and pGALRE-luc containing PPARα-LBD, PPARβ/δ-LBD, or PPARγ-LBD. pProEX-PPAR and pET-28a were used for purification of His-tagged recombinant hPPAR-LBD protein.

### 2. Cell culture and transfection

A HEK293 cell line was cultured in Dulbecco's Modified Eagle Medium (DMEM) containing 10% Fetal Bovine Serum (FBS) and 1% penicillin-streptomycin (P/S).

A mouse preadipocyte 3T3-L1 cell line was cultured in DMEM containing 10% goat serum (CS) and 1% P/S. For differentiation of 3T3-L1, 10% FBS and 1% P/S were used. The cells were cultured in a 37°C incubator humidified with 5% CO₂ atmosphere.

For gene transfection, microporation was performed on HEK293 cells using an MP-100 microporator (Invitrogen, Carlsbad, CA, USA).

### 3. Luciferase assay

Transfected HEK293 cells were treated with a drug and after 24 hours, the cells were collected, resuspended in a luciferase lysis buffer (Promega, Madison, Wisconsin, USA), and incubated on ice for 10 minutes. The cell lysate was removed by centrifugation at 15,000 rpm for 10 minutes at 4°C, and then the supernatant was separated. Next, Firefly Luciferase (Fluc) and Renilla Luciferase (Rluc) activities were measured using the Dual Luciferase Reporter Assay System (Promega, Madison, Wisconsin, USA). The activity ratio of Fluc to Rluc was defined as the Ligand Binding Domain (LBD) activity of PPAR.

### 4. CNBr-bead conjugation assay

100 mg of CNBr-activated Sepharose 4B was added to an e-tube, and then the beads were activated by rotating with 1 ml of 1 mM HCl for 10 minutes at room temperature. After 10 minutes, the solution was newly replaced and this process was repeated. The supernatant was removed by centrifugation at 5,000 rpm for 1 minute, and then the sample was washed with a coupling buffer, which was repeated three times. The sample was separated with 20 mg beads in another e-tube. Each e-tube was added with the drug and rotated overnight at 4°C, at which time the concentration was measured to be 20 µM. The next day, the sample was washed three times with a coupling buffer. After washing, the sample was blocked with 1 ml of a blocking buffer for 2 hours at room temperature. After blocking, the sample was washed three times with a washing buffer and repeated in the order of a washing buffer and a pull-down buffer. Next, the supernatant was centrifuged at 5,000 rpm for 1 minute to remove the supernatant. The pellet was added with 0.8 ml of pull-down buffer and purified protein and rotated overnight at 4°C. Finally, the sample was washed three times with a Pull-down buffer. The supernatant was completely removed, and the pellet was detected by Western blotting.

### 5. In silico docking analysis

Crystal structures (PPARα: 4BCR, PPARβ/δ: 5U46, PPARγ: 5YCP) of LBDs of human PPAR family were prepared through the RCSB Protein Data Bank. For further docking analysis, the energy of D6 or PPAR ligands (WY14643, GW501516, and rosiglitazone) was minimized using the open babel within PyRx software.

### 6. 3T3-L1 adipocyte differentiation

3T3-L1 cells were seeded in a 12-well plate at a density of 5 × 10⁴ cells/well. The cells were cultured until confluency was achieved in DMEM containing 10% CS and 1% P/S. On day 2 after achieving 100% confluency, the cells were added with a DMEM medium containing 10% FBS and 1% P/S supplemented with MDI (0.5 mM IBMX, 1 µM dexamethasone, 1 µg/ml insulin). On day 2 of MDI addition, the medium was replaced with 1 µg/ml insulin.

After 2 days, the medium was replaced with DMEM containing 10% FBS and 1% P/S, and treated with a drug. The medium was replaced with fresh DMEM containing 10% FBS and 1% P/S, and treated with the drug three times every 2 days. Finally, the medium was replaced with DMEM containing only the drug in a serum-free state (serum starvation). After 2 days, differentiation of 3T3-L1 was completed.

### 7. Oil Red O staining

After the differentiation of 3T3-L1 in the 12-well plate was completed, the cells were fixed with 4% paraformaldehyde and stained with an Oil Red O solution, and then the stained samples were photographed under a microscope (Nikon Eclipse Ti-s). An Oil Red O dye of the samples was dissolved in isopropanol, detected by a spectrophotometer at 492 nm, and the lipid droplet size distribution was measured using ImageJ. OCT-embedded liver samples were stained according to the Roy Ellis protocol.

### 8. Western blotting

Differentiated 3T3-L1 cells were treated with a drug, and then the cells were harvested and resuspended in a PRO-PREP^{™} Protein Extraction Solution (iNtRON Biotechnology, Korea). The samples were incubated on ice for 10 minutes, vortexed for 2 seconds, and then incubated on ice again for 10 minutes. After centrifugation (15,000 rpm, 20 minutes, 4°C), the protein concentration was measured by the Bradford assay using 1 mg/ml BSA. Equal amounts of proteins were separated on an SDS-polyacrylamide gel and blotted onto a polyvinylidene difluoride membrane (PVDF) (Millipore, Billerica, MA, USA). After blocking with a blocking solution (5% BSA in TTBS) at room temperature for 30 minutes, the samples were incubated with primary antibodies (anti-HSL, anti-ATGL, anti-Actin, etc.) overnight at 4°C. Thereafter, the samples were incubated with secondary antibody for 2 hours at room temperature and the signals were detected with LAS4000.

### 9. Experimental animals

The care and use of experimental animals were carried out with the approval of the Institutional Animal Care and Use Committee of Pohang University of Science and Technology. C57BL/6J mice were purchased from Japan SLC, Inc. 50 four-week-old male C57/BL/6J mice were acclimated for 2 weeks. The mice were randomly divided and orally administered with vehicle or 5 mg/kg/day of D6 or WY14643 for 13 weeks (oral gavage), and obesity was induced by a 60% high-fat diet (#D12492, Research diets, NJ, USA). Body weights and food intakes were measured weekly from 6 weeks of age, and after 16 weeks of age, adipose tissue and muscle volumes were measured using a Trimodality imaging system.

### 10. Intraperitoneal glucose tolerance test (IP-GTT) and intraperitoneal insulin tolerance test (IP-ITT)

A glucose tolerance test (GTT) was performed on mice after 16 weeks of age after fasting for 16 hours and intraperitoneal glucose administration at 2 g/kg body weight. The blood glucose levels were measured at 0, 15, 30, 45, 60, 90, and 120 minutes after tail vein injection.

An insulin tolerance test (ITT) was performed on mice after 17 weeks of age after fasting for 3 hours and intraperitoneal insulin administration at 0.75 U/kg body weight. The blood glucose levels were measured at 0, 15, 30, 45, 60 and 90 minutes after tail vein injection.

### 11. Biopsy

Mouse tissues were fixed with 10% formalin solution (Sigma-Aldrich, St. Louis, MO, USA), and paraffin-embedded tissues were segmented and stained with hematoxylin and eosin (H&E) staining and Sirius Red staining (IHC world, Woodstock, USA).

Fibrosis and vesicular fat in liver tissue were observed using Sirius Red and Oil Red O staining methods. The NAFLD activation score was evaluated by referring to Kleiner, D. E. et al. Hepatology 41, 1313-1321, doi:10.1002/hep.20701 (2005), etc., according to the NASH Clinical Research Network Scoring System.

### 12. Serum analysis

Mouse blood was obtained by cardiac puncture and incubated at room temperature for 30 minutes. Next, the blood sample was centrifuged at 3,000 rpm for 30 minutes at 4°C, and then the supernatant was collected and analyzed.

### <Examples>

### Example 1. Confirmation of pan-PPAR agonist effect

To confirm whether a compound represented by Chemical Formula 1 was a PPAR agonist, Gal4 trans-activated luciferase assay was performed. Specifically, HEK293 cells were transfected with the Gal4-luciferase reporter systems of PPARα, PPARβ/δ, and PPARγ, respectively, harvested after drug treatment for 24 hours, and then analyzed by Dual-Luciferase assay. Relative luciferase activity was compared as Fluc activity to Rluc activity. WY14643, GW501516, and rosiglitazone were representative PPARα, PPARδ, and PPARγ agonists, respectively, and serve as positive controls.

Experimental results showed that the compound represented by Chemical Formula 1 dose-dependently increased the relative luciferase activity for all PPARα, PPARβ/δ, and PPARγ (FIG. 1).

Meanwhile, in order to confirm whether the compound represented by Chemical Formula 1 directly bound to the PPAR protein, CNBr-bead conjugation assay was performed. Specifically, the compound represented by Chemical Formula 1 (D6) and each PPAR agonist (WY14643, GW501516, and rosiglitazone) were coupled with CNBr and reacted with His-tagged recombinant human PPAR-Ligand Binding Domain (hPPAR-LBD), and then the conjugation to PPAR was confirmed by Western blotting. A free drug ("Free"), which was not conjugated to CNBr, served as a competitor to the CNBr-coupled drug. When the CNBr-coupled drug that reacted with hPPAR-LBD was mixed with the free drug, the band disappeared.

Experimental results showed that the compound represented by Chemical Formula 1 was conjugated to all three subtypes of PPAR as much potently as a PPAR agonist used as a positive control.

To further support the experimental results, in-silico analysis was performed to predict the binding position of the compound represented by Chemical Formula 1 within a ligand-binding domain (LBD) of PPAR and the binding energy between the PPAR LBD and the compound represented by Chemical Formula 1. As a result, the binding energy of the compound represented by Chemical Formula 1 of the present invention was confirmed to be at a similar level to that of representative agonists of PPAR.

### Example 2. Confirmation of effects on energy metabolism, including lipid accumulation in adipocytes

To determine the effect of the compound represented by Chemical Formula 1 on adipocytes, a 3T3-L1 adipocyte differentiation experiment was performed. After treatment with the compound represented by Chemical Formula 1 (D6), rosiglitazone (Ro), or both (drug concentration: 25 µM) during differentiation, metabolic changes such as the accumulation of lipid droplets within adipocytes were observed using Oil Red O staining (FIG. 3). As a result, it was confirmed that the lipid droplets in adipocytes treated with the compound represented by Chemical Formula 1 alone were similar to those in a DMSO-treated group, which was a negative control group, and that the lipid accumulation increased by rosiglitazone treatment was reduced when treated together with the compound represented by Chemical Formula 1 (left sides of FIGS. 3 and 4). In particular, when rosiglitazone was treated alone, the size of small lipid droplets decreased and the size of large lipid droplets increased, but when the compound represented by Chemical Formula 1 was treated together, it was confirmed that the size distribution of lipid droplets was effectively reduced (right side of FIG. 4).

### Example 3. Confirmation of effect on energy metabolism in high-fat diet mice

To confirm a metabolic disease inhibitory effect of the compound represented by Chemical Formula 1 through animal experiments, mice were acclimated for 2 weeks, and then obesity was induced by feeding a 60% high fat diet (HFD) and orally administered with vehicle or 5 mg/kg/day of the compound represented by Chemical Formula 1 (D6) or WY14643 for 13 weeks. Experiments such as IP-GTT, IP-ITT, and micro-CT were performed at the week 10 of drug administration (FIG. 5).

As a result of the experiment, when the compound represented by Chemical Formula 1 was administered to high-fat diet mice (HFD-D6), there was no significant change in food intake compared to a control group (HFD-V), but the rate of weight gain was significantly reduced (FIG. 6).

In addition, as micro-CT imaging and quantification results in high-fat diet mice, the volume of adipose tissue in mice administered with the compound represented by Chemical Formula 1 was significantly reduced compared to the control group (FIGS. 7 and 8).

Meanwhile, as a result of measuring the organ weights of high-fat diet mice, the epididymal white adipose tissue (eWAT) and liver weights were reduced in mice administered with the compound represented by Chemical Formula 1 (HFD-D6) compared to mice not administered with the drug (HFD-V) (FIG. 9).

To determine the effect of the compound represented by Chemical Formula 1 on energy metabolism, a glucose tolerance test (GTT) and an insulin tolerance test (ITT) were performed. As a result of the experiment, it was confirmed that glucose tolerance and insulin resistance were improved in high-fat diet mice (HFD-D6) administered with the compound represented by Chemical Formula 1, thereby improving metabolic diseases such as diabetes-related symptoms (FIGS. 10 and 11).

As a result of analysis of mouse serum components, it was confirmed that blood sugar and blood lipids (LDLC) were significantly reduced in mice administered with the compound represented by Chemical Formula 1 to have an effect of improving metabolic diseases such as diabetes and dyslipidemia (FIGS. 12 and 13).

### Example 4. Histological observation on adipose tissues of high-fat diet mice

To observe the effects of the compound represented by Chemical Formula 1 on metabolic diseases, biopsy was performed on adipose tissues of high-fat diet mice.

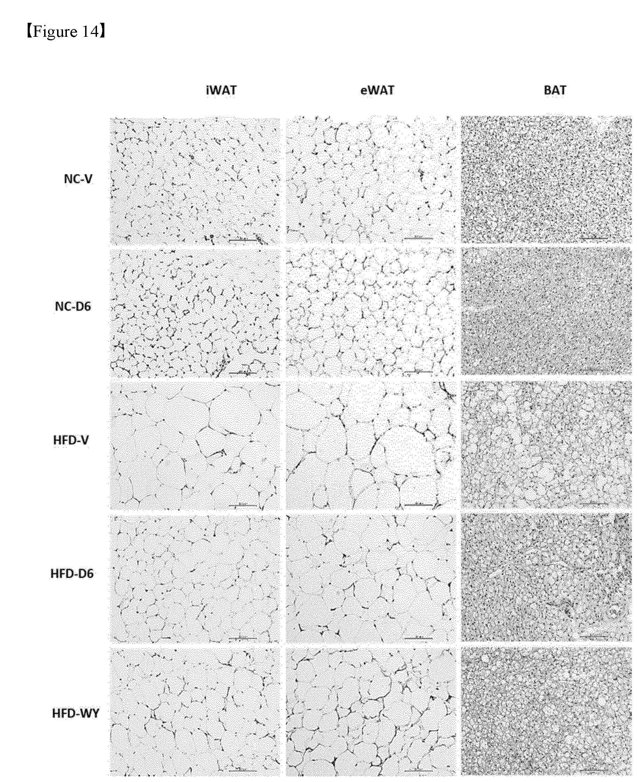

H&E staining results for mouse adipose tissue showed that the adipocyte sizes were reduced in high-fat diet mice (HFD-D6) administered with the compound represented by Chemical Formula 1 (FIG. 14). As a result of quantitatively measuring the adipocyte size distribution, the effects of reducing adipocyte sizes in inguinal WAT (iWAT), epididymal white adipose tissue (eWAT), and brown adipose tissue (BAT) were confirmed (FIG. 15).

### Example 5. Histological observation on liver tissues of high-fat diet mice

To evaluate the effects on fatty liver-related symptoms after administration of the compound represented by Chemical Formula 1, H&D, Sirius Red, and Oil Red O staining were performed on liver tissues of mice fed with a high-fat diet for 13 weeks.

As a result of the experiment, fat accumulation, cell ballooning (arrow), and the like were observed in the liver tissues of high-fat diet mice (HFD-V), and histological findings of fatty liver-related diseases were reduced in high-fat diet mice (HFD-D6) administered with the compound represented by Chemical Formula 1 (FIG. 16).

More specifically, as a result of evaluating the NAFLD activity score through H&E images of liver tissue, it was confirmed that the high-fat diet mice (HFD-D6) administered with the compound represented by Chemical Formula 1 showed a better effect of improving the NAFLD disease index than the high-fat diet mice (HFD-WY) administered with WY14643, which was a positive control group (FIG. 17). In addition, as a result of quantitatively evaluating the level of lipid accumulation in the liver using Oil Red O, it was confirmed that the level of lipid droplet accumulation was reduced in high-fat diet mice (HFD-D6) administered with the compound represented by Chemical Formula 1 (FIG. 18).

It will be appreciated by those skilled in the art that the present invention as described above may be implemented into other specific forms without departing from the technical spirit thereof or essential characteristics. Thus, it is to be appreciated that embodiments described above are intended to be illustrative in every sense, and not restrictive. The scope of the present invention is represented by claims to be described below rather than the detailed description, and it is to be interpreted that the meaning and scope of the claims and all the changes or modified forms derived from the equivalents thereof come within the scope of the present invention.

## Claims

1. A pharmaceutical composition for use in preventing or treating metabolic diseases, comprising a compound represented by the following Chemical Formula 1 or a salt thereof as an active ingredient.

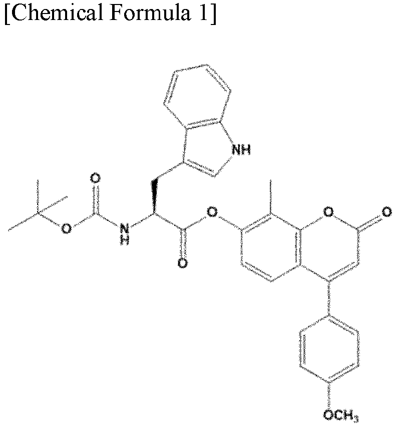

2. The pharmaceutical composition for use of claim 1, wherein the compound represented by Chemical Formula 1 or the salt thereof activates PPARα, PPARβ/δ, and PPARγ.

3. The pharmaceutical composition for use of claim 1, wherein the metabolic disease is at least one selected from the group consisting of obesity, diabetes, fatty liver diseases, dyslipidemia, cardiovascular diseases, and metabolic syndrome.

4. A food composition for use in improving metabolic health comprising a compound represented by Chemical Formula 1 or a salt thereof.

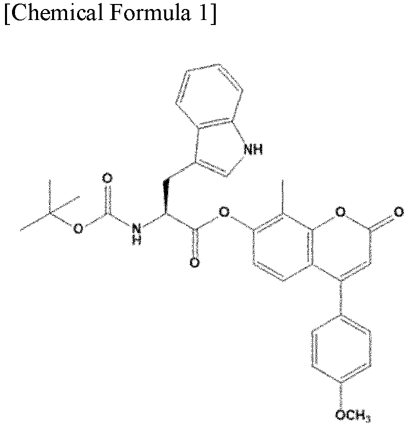

5. The food composition for use of claim 4, wherein the improvement of the metabolic health is at least one effect selected from the group consisting of improvement in obesity, drop in blood sugar, improvement in fatty liver, improvement in lipid metabolism, improvement in blood circulation, and improvement in metabolic syndrome.

6. A feed composition for use in improving metabolic health comprising a compound represented by Chemical Formula 1 or a salt thereof.
